# EUROPEAN PATENT SPECIFICATION

(11) **EP 2 415 475 B1**
(45) Date of publication and mention of the grant of the patent: **31.12.2014**
(21) Application number: 10759105.9
(22) Date of filing: 23.04.2010
(51) Int. Cl.: A61K 45/06, A61K 31/047, A61K 31/4439, A61K 31/7016, A61K 31/702, A61K 31/715, A61K 31/732, A61K 31/733

(54) **PHARMACEUTICAL COMPOSITION COMPRISING A PROTON PUMP INHIBITOR AND A PREBIOTIC FOR THE TREATMENT OF ULCEROUS LESIONS OF THE STOMACH AND DUODENUM**
PHARMAZEUTISCHE ZUSAMMENSETZUNG MIT EINEM PROTONENPUMPENHEMMER UND EINEM PRÄBIOTIKUM ZUR BEHANDLUNG EITERNDER WUNDEN IM MAGEN UND IM ZWÖLFFINGERDARM
COMPOSITION PHARMACEUTIQUE COMPRENANT UN INHIBITEUR DE LA POMPE À PROTONS ET UN PRÉBIOTIQUE POUR LE TRAITEMENT DES ULCÈRES DE L'ESTOMAC OU DU DUODÉNUM

(30) Priority: 30.03.2009 RU 2009111362
(43) Date of publication of application: 08.02.2012
(73) Proprietor: Dikovskiy, Aleksander Vladimirovich, Moscow 123182 (RU)
(72) Inventor: DOROZHKO, Oleg Valentinovich, Moscow 115446 (RU)
(74) Representative: Duncan, Garreth Andrew
(86) International application number: PCT/RU2010/000197
(87) International publication number: WO 2010/114425

(56) References cited:
- WO-A1-94/03184
- WO-A1-94/18986
- WO-A1-98/23272
- WO-A1-2004/093875
- WO-A1-2007/117175
- RU-C1- 2 325 187
- RU-C2- 2 184 558
- US-A- 5 514 660
- US-A1- 2004 018 085
- US-A1- 2004 180 850
- SIMON P M ET AL: "Inhibition of helicobacter pylori binding to gastrointestinal epithelial cells by sialic acid-containing oligosaccharides", INFECTION AND IMMUNITY, AMERICAN SOCIETY FOR MICROBIOLOGY, WASHINGTON; US, vol. 65, no. 2, 1 January 1997 (1997-01-01), pages 750-757, XP002972942, ISSN: 0019-9567

## Description

The invention relates to medicine and pharmacology, more particularly to a medicinal product - pharmaceutical composition for treatment of gastric and duodenal ulcer.

The gastric and duodenal ulcer is a chronic recurrent disease defined as mucosal erosions in the stomach and the duodenum. The disease relates to the most common lesions of organs of gastrointestinal tract. According to the epidemiologic evidence as many as 8-10 % of the adult population of the world has peptic ulcer. Less than 1 % of the child population has peptic ulcer. People young and middle age has peptic ulcer more often. Ratio of gastric ulcer to duodenal ulcer is 1 to 4, and duodenal ulcer is observed mainly at young age, for the middle and elderly age - frequency of gastric ulcer increases. Duodenal ulcer is observed at men considerably more often.

Clarification of a role of Helicobacter pylori in peptic ulcer development for the first time allowed of definitely speaking not only about a pathogenesis, but also about a disease etiology. The epidemiologic evidence obtained in the various countries shows that in 100 % of cases of duodenal ulcer connection between the disease and Helicobacter infection is revealed.

According to the modern knowledge regardless of stage of disease (exacerbation or remission) it is required a course of eradication therapy with germicides for each patient if Helicobacter pylori strain is isolated from the patient. In overwhelming majority of the cases eradication is fulfilled in a stage of the peptic ulcer exacerbation when endoscopic detection of ulcer is accompanied by confirmation of H. pylori in the mucous membrane of the stomach (by morphological or urease method).

Last years serious problems related to growing antibacterial resistance of Helicobacter pylori strains, first of all to metronidazole (more than in 30% of cases) and clarithromycin (more than in 10% of cases) were detected when carrying out eradication therapy. From the practical aspect, it means essential decrease of eradication efficiency when using regimen that included the specified antibacterial drug products. So, for example, in case of resistance of H. pylori to metronidazole efficiency of the regimens included clarithromycin and metronidazole, amoxicillin and metronidazole decreased from 93% and 91% to 76% and 61% respectively. When using regimen that included clarithromycin, eradication rate decreased to 44-69% in patients with clarithromycin-resistant strains.

Modern treatment regimen for patients with peptic ulcer combines antimicrobial eradication therapy with antisecretory therapy included proton pump inhibitor (PPI). At present two generations of PPI are known. The first generation of the medicines (omeprazole, pantoprazole, lansoprazole and rabeprazole) possesses approximately identical ability to suppress gastric acid secretion. Proton pump inhibitor has the most powerful effect among all antisecretory agents. This fact explains the leading position of this class of medicinal products in treatment of acid-related diseases and H. pylori-associated diseases.

Omeprazole is the first medicine from group of proton pump inhibitors, synthesized in 1979 in Sweden (Losec). Last 10 years many countries consider omeprazole to be agent of choice in treatment of acid-related diseases (as monotherapy or in combination with antibiotics).

Leader of the second generation esomeprazole is isolated from racemic mixture of the right- (R-) and left-handed (S-) isomers of omeprazole. Esomeprazole more effectively suppresses gastric acid secretion in healthy volunteers as well as in patients with reflux esophagitis. Esomeprazole kept pH value < 4 in stomach by 10-15 % above than PPI of the first generation. In spite of the differences in structure of proton pump inhibitors clinical efficiency of these medicines in therapy of gastroesophageal reflux disease becomes almost equivalent on the 7-8th day of administration.

At present possibilities of pharmacotherapy of peptic ulcer considerably increased. After proton pump inhibitors were introduced into clinical practice a physician has less problems with cicatrization of gastric and duodenal ulcers when treating patients with acute condition of peptic ulcer, and eradication therapy permits considerable decrease of frequency of relapses of peptic ulcer.

The medicinal product which contains a combination of proton pump inhibitor and antacid agent is known. Proton pump inhibitor and antacid rafting agent are administrated simultaneously, but as separate dosage forms. This method supposes the treatment regimen that is badly observed by the patients because of the large quantity of daily doses. Besides, there are additional problems with observance of this regimen when proton pump inhibitor and antacid are administrated at various times and they are different galenical medicinal products (WO 98/23272). Administration of two or even more than two various dosage forms is inconvenient for patient or insufficient to obtain optimal results.

Antimicrobial drug product, containing a preparation from group of taurolidine, taurultam or their combinations as a remedy for gastrointestinal tract disturbance of mammals caused by Helicobacter pylori, Helicobacter heilmannii or association of this microorganisms, is known. There is a method of treatment of relevant diseases and compositions based on the combination of taurolidine, taurultam or their combination with medicines that forms protective coat on patient's mucous coat of the stomach, and compositions based on the combination of taurolidine, taurultam or their combination with proton pump inhibitor. The invention not only makes it possible to suppress growth of this campylobacter species but also blocks their linkage with epithelial cells of mucous coat of the stomach so the bacteria become nonpathogenic and fail to release cytotoxin VacA (RU No 2227033).

Disadvantage of this drug product is a toxic action of antimicrobials and intestinal dysbacteriosis as a result of suppression of normal microflora by antimicrobials.

The antimicrobial drug product containing inhibitor H⁺K⁺ATPase (proton pump inhibitor), synergistic combination of three antimicrobials is known, and the treatment regimen additionally includes from first day alive microbial lactobacillus and bifidobacterium cultures having a titer of 10¹⁰-10¹² per 1 ml in a daily (single) dose of 5 ml, duration of 28 days (RU No. 2184558, prototype).

Disadvantage of this drug product is a toxic action of significant dose of antimicrobials and absence of stable treatment result since heterologous strains of lactobacillus and bifidobacterium within several days are eliminated from new host's gastrointestinal tract and also quickly dies in liquid culture at storage.

Technical object that is solved by this invention is the development of the effective medicinal product for treatment of gastric and duodenal ulcer and expansion of an arsenal of drug products for the specific therapy.

Technical result that provides a solution of the problem is the attainment of accelerated reparation of mucosal erosions in the stomach and effective stable eradication of H. pylori especially from mucous coat of the duodenum. In trial groups of patients the eradication is up to 95-100% without antibacterial therapy.

The essence of the invention referred to a composition lies in the fact that the pharmaceutical composition for treatment of gastric and duodenal ulcer includes at least one PPI and at least one prebiotic according to claim 1 with following content of the composition components in mass %:
- proton pump inhibitor of 0.05-25%;
- prebiotic of 40-95%;
- excipients up to 100%.

The composition contains prebiotic from the group of di- and trisaccharides: lactulose, lactosucrose, melibiose, xylobiose, stachyose, raffinose, or contains prebiotic from the group of oligosaccharides: fructooligosaccharide, galactooligosaccharide, maltooligosaccharide, xylooligo-saccharide, isomaltooligosaccharide, gentioligosaccharide, or contains prebiotic from the group of polysaccharides: arabinogalactan, pectin, pullulan, inulin, lignin. In special cases of realization the composition contains proton pump inhibitor from the group: omeprazole, pantoprazole, lansoprazole, rabeprazole and esomeprazole. The composition contains PPI omeprazole of 10-120 mg or it contains PPI pantoprazole of 20-800 mg or it contains PPI lansoprazole of 10-600 mg, or it contains PPI rabeprazole of 10-200 mg or it contains PPI esomeprazole of 20-240 mg. Preferentially the dosage form of the composition: suspension for oral administration, solution for oral administration, capsules, tablets, powders, sachets, pellets, granules, etc.

The essence of the invention referred to a method lies in the fact that due to the declared treatment method of gastric and duodenal ulcer when the eradication of H. pylori is attained during the course of therapy in patients (at least once a day, before food intake, at least, within 14 days) with enteral administration of the pharmaceutical composition of PPI and prebiotic, developed according to any of claims 1-5, 6-11. Thus, eradication of H. pylori is attained without administration of antibiotics during the course of therapy.

The role of prebiotic (for example, lactulose, fructooligosaccharide, etc.) for attainment of technical result consists in active stimulation of growth of patient's own lactobacilli in the duodenum and competitive inhibition of growth of Helicobacter pylori, that is a major factor of the causative agent eradication. After administration of the medicinal product patient's own lactobacilli are bred actively (its overgrowth arises) in the stomach and duodenum, and growth and reproduction of H. pylori is depressed even without antimicrobial therapy by antibiotics.

Prebiotic is a non-digestible food ingredient that beneficially affects the host health by selectively stimulating the growth and/or metabolic activity of one or a limited number of bacteria in the colon (Gibson G.R., Roberfroid M.B., 1995). Dietary fibers cannot be digested by the digestive enzymes of the small intestine and pass to the colon without any changes. In the lower colon oligosaccharides are fermented by bifidobacteria and lactobacilli. It leads to the growth of the total bacterial mass, stimulation of immune system, increase of the intestinal motility and further normalization of functional activity of the gastrointestinal tract.

### Examples of the Practical Realization of the Invention

As an example of practical realization of the declared invention the results of the clinical trials of the pharmaceutical composition for treatment of gastric and duodenal ulcer, containing PPI and prebiotic as active components.

These examples of realization of the declared purpose confirm synergistic effect of PPI and prebiotic - growth-promoting factor of the patient's own normal microflora - in patients with gastric and duodenal ulcer.

There were enrolled 80 patients aged 19 to 57 years in the study: males and females with newly diagnosed gastric and duodenal ulcer, five from them with Zollinger-Ellison syndrome.

All patients were divided into 8 groups of 10 patients each, 4 groups of them were experimental: the declared dosage forms on the base of PPI and prebiotic were orally administered by them. For the first group active components of the composition were omeprazole and a lactulose; for the second group - pantoprazole and prebiotic lactitol (comparative); for the third group - lansoprazole, and prebiotic inulin; for the fourth group - rabeprazole and prebiotic from group of amino acids (glutamic acid; comparative). 5^{th}, 6^{th}, 7^{th} and 8^{th} groups were control: patients of the 5^{th} group received monotherapy by omeprazole; patients of the 6^{th} group - by lansoprazole; patients of the 7^{th} group - esomeprazole and patients of the 8^{th} group received standard therapy for the elimination of H. pylori: omeprazole, metronidazole and clarithromycin.

All patients received treatment according to the similar regimen: took the composition of PPI and prebiotic 1-2 times a day within 14 days.

The following therapeutic doses of PPI were included in the pharmaceutical composition:

| **Proton Pump Inhibitor** | **Daily therapeutic dose (mg)** |
|---|---|
| 1^{st} generation | |
| **Omeprazole,** | 20- 120^{*)} |
| **Pantoprazole,** | 40-80 |
| **Lansoprazole and** | 15-60 |
| **Rabeprazole** | 10-20 |

| 2^{nd} generation | |
|---|---|
| **Esomeprazole** | 20-40 |

| | |
|---|---|
| ⁺⁾ doses are presented on the basis of the maximum therapeutic doses recommended for patients with Zollinger-Ellison syndrome | |

Patients with gastric and duodenal ulcer were diagnosed on the basis of the objective clinical data (the complaint of pain, vomiting, bleeding in past history and discomfort in the epigastric area) and laboratory researches (examination of the mucosa by the esophagogastroduodenoscopy).

Modern microbiological, biochemical, immunological and radiological methods were used to diagnose Helicobacter infection:
1. Cytological and histological examination of imprint smear of the biopsy material of the gastric antrum mucosa, obtained by gastroscopy. Thus, microcolonies of Helicobacter pylori are detected using special stain.
2. Urease test. The biopsy material of the gastric mucosa was examinated for the presence of the urease-enzyme specific for Helicobacter pylori **(Helicobacter Test INFAI).**
3. C-urea breath test. Patient swallow urea labeled with a short-lived radioactive isotope carbon-13. Under the action of H. pylori urease urea is converted into ammonia and carbon dioxide. Carbon dioxide contains radioactive isotope and it is exteriorized by lungs that allows detecting its concentration in exhaled air.
4. Microbiological method. Inoculation and isolation of pure culture of H. pylori from the biopsy material of the mucosa, obtained by gastroduodenoscopy.
5. Immunological method. Revealing of antibodies specific to H. pylori antigens in serum.
6. Detection of Helicobacter pylori by the polymerase chain reaction (PCR). At present it is the most exact method for diagnosis of H. pylori infection, especially in case of the morphological transformation of the bacteria to a coccal form (for example, after a course of antibacterial therapy) and when other diagnostic methods (for example, urease test) yield false-negative results.

The evidence of the presence of H. pylori infection has important value for the patient, since successful treatment of peptic ulcer is possible only after elimination of this microorganism from the gastrointestinal tract (eradication).

Before the beginning and after the termination of the eradication therapy all 80 patients have been enrolled in the clinical trials were microbiologically tested on the presence of H. pylori, lactobacilli and bifidobacteria in the stomach and duodenum content.

The material was analyzed in microbiological laboratory of the Pasteur Institute of Epidemiology and Microbiology, St. Petersburg.

Strains of H. pylori were identified on the base of analysis of colony and cell morphology, cultural, biochemical and tinctorial properties of microorganisms. Lactobacillus strains isolated in the material from the intestine additionally were identified on catalase and oxidase activity.

The bacterium quantity in the material was estimated by calculation of colonies on dense nutrient mediums that were represented in the form of Ig CFU/ml.

The result of the clinical trials were confirmed that the pharmaceutical composition possesses high therapeutic efficacy in relation to the causative agent of gastric and duodenal ulcer Helicobacter pylori (see table 1 - 4) and affects the duodenum microflora (stimulates growth of lactobacilli) that is a crucial factor of the therapy efficiency. Efficiency of the declared composition essentially exceeded efficiency of the control PPI preparations used as monotherapy (see table 5-7) and as standard eradication therapy (see table 8) which besides PPI included antibiotics (clarithromycin or amoxicillin) in combination with metronidazole (the Maastricht 2 - 2000 Consensus Report).

All patients within 12 months after the treatment termination were observed in the outpatient setting. The disease relapses were not noted in groups of the patients who took the declared compositions of PPI and prebiotics (1 - 4).

**Table 1.**

| Laboratory Examination Results. Patients of the 1^{St} group | | |
|---|---|---|
| **Laboratory Test** | **Peptic Ulcer, n=10** | |
| | **Before therapy** | **After therapy** |
| Isolation of Helicobacter pylori in pure culture | **100 %** | **0 %** |
| Cytological test of the biopsy material of the gastric mucosa | **70 %** | **0 %** |
| Urease test | **60 %** | **10 %** |

**Table 2.**

| Laboratory Examination Results. Patients of the 2^{nd} group (comparative) | | |
|---|---|---|
| **Laboratory Test** | **Peptic Ulcer, n=10** | |
| | **Before therapy** | **After therapy** |
| Isolation of Helicobacter pylori from stomach content | **100 %** | **10 %** |
| Cytological test of the biopsy material of the gastric mucosa | **60 %** | **0 %** |
| Urease test | **70 %** | **10 %** |

**Table 3.**

| Laboratory Examination Results. Patients of the 3^{rd} group | | |
|---|---|---|
| **Laboratory Test** | **Peptic Ulcer, n=10** | |
| | **Before therapy** | **After therapy** |
| Isolation of Helicobacter pylori from stomach content | **100 %** | **0 %** |
| Cytological test of the biopsy material of the gastric mucosa | **70 %** | **20 %** |
| Urease test | **60 %** | **0 %** |

**Table 4.**

| Laboratory Examination Results. Patients of the 4^{th} group (comparative) | | |
|---|---|---|
| **Laboratory Test** | **Peptic Ulcer, n=10** | |
| | **Before therapy** | **After therapy** |
| Isolation of Helicobacter pylori from stomach content | **100 %** | **0 %** |
| Cytological test of the biopsy material of the gastric mucosa | **60 %** | **0 %** |
| Urease test | **60 %** | **10 %** |

**Table 5.**

| Laboratory Examination Results. Patients of the 5^{th} group | | |
|---|---|---|
| **Laboratory Test** | **Peptic Ulcer, n=10** | |
| | **Before therapy** | **After therapy** |
| Isolation of Helicobacter pylori from stomach content | **100 %** | **50%** |
| Cytological test of the biopsy material of the gastric mucosa | **80 %** | **40 %** |
| Urease test | **70 %** | **30 %** |

**Table 6.**

| Laboratory Examination Results. Patients of the 6^{th} group | | |
|---|---|---|
| **Laboratory Tests** | **Peptic Ulcer, n=10** | |
| | **Before therapy** | **After therapy** |
| Isolation of Helicobacter pylori from stomach content | **100 %** | **60 %** |
| Cytological test of the biopsy material of the gastric mucosa | **60 %** | **40 %** |
| Urease test | **70 %** | **60 %** |

**Table 7.**

| Laboratory Examination Results. Patients of the 7^{th} group | | |
|---|---|---|
| **Laboratory Tests** | **Peptic Ulcer, n=10** | |
| | **Before therapy** | **After therapy** |
| Isolation of Helicobacter pylori from stomach content | **100 %** | **50 %** |
| Cytological test of the biopsy material of the gastric mucosa | **70 %** | **40 %** |
| Urease test | **60 %** | **40 %** |

**Table 8.**

| **Laboratory Examination Results. Patients of the 8^{th} group** | | |
|---|---|---|
| **Laboratory Tests** | **Peptic Ulcer, n=10** | |
| | **Before therapy** | **After therapy** |
| Isolation of Helicobacter pylori from stomach content | **100 %** | **30 %** |
| Cytological test of the biopsy material of the gastric mucosa | **70 %** | **20 %** |
| Urease test | **60 %** | **30 %** |

Thus the results of clinical trials objectively presents the efficiency of treatment of gastric and duodenal ulcer by oral administration of the declared pharmaceutical composition which contains PPI and selected prebiotic as active components. The components ensure synergistic effect expressed in accelerated reparation of mucosal erosions in the stomach and successful eradication of H. pylori at the expense of colonization of the duodenum by lactobacilli and competitive inhibition of H. pylori. According to endoscopic control the therapeutic effect continuous for a long time and does not accompanied with relapses within 12 months during monitoring of patient. Relapses of disease in patients of control groups were registered in 35% of cases within first 6 months of supervision.

Complex therapy of the peptic ulcer associated with Helicobacter infection by pharmaceutical composition of PPI and selected prebiotic - in the condition of high stomach content pH - ensures to stimulate actively growth of lactobacilli in the upper gastrointestinal tract including duodenum and to enlarge essentially titer of lactobacilli - antagonists to H. pylori that essentially raises efficiency of antiulcerous therapy.

High clinical efficiency and safety caused by synergism of PPI and selected prebiotic action in the upper gastrointestinal tract, absence of side effects and relapses and compliance of the combined therapy with declared pharmaceutical composition testify that the declared formulation is a new promising drug product for treatment of gastric and duodenal ulcer.

## Claims

1. Pharmaceutical composition for treatment of gastric and duodenal ulcer includes at least one PPI and at least one prebiotic with following content of the composition components in weight %:
- proton pump inhibitor of 0.05-25%;
- prebiotic of 40-95%;
- excipients up to 100%,
wherein the prebiotic is selected from a group of
a di- or trisaccharide selected from the group of lactulose, lactosucrose, melibiose, xylobiose, stachyose and raffinose; and/or
an oligosaccharide selected from the group of fructooligosaccharide, galactooligosaccharide, maltooligosaccharide, xylooligosaccharide, isomaltooligosaccharide and gentioligosaccharide; and/or
a polysaccharide selected from the group of arabinogalactan, pectin, pullulan, inulin and lignin.

2. Pharmaceutical composition of claim 1, **characterized in that**, it contains proton pomp inhibitor from the group: omeprazole, pantoprazole, lansoprazole, rabeprazole and esomeprazole.

3. Pharmaceutical composition of claim 2, **characterized in that**, it contains PPI omeprazole of 10-120 mg.

4. Pharmaceutical composition of claim 2, **characterized in that**, it contains PPI pantoprazole of 20-800 mg.

5. Pharmaceutical composition of claim 2, **characterized in that**, it contains PPI lansoprazole of 10-600 mg.

6. Pharmaceutical composition of claim 2, **characterized in that**, it contains PPI rabeprazole of 10-200 mg.

7. Pharmaceutical composition of claim 2, **characterized in that**, it contains PPI esomeprazole of 20-240 mg.

8. Pharmaceutical composition according to any one of claims 1 to 7, **characterized in** the dosage form of the composition being a suspension for oral administration, a solution for oral administration, or capsules, tablets, powders, sachets, pellets, granules for oral administration.

9. Pharmaceutical composition according to any one of claims 1 to 8 for use in the treatment of gastric and duodenal ulcer when the eradication of H. pylori is attained during the course of therapy in patients (at least once a day, before food intake, at least, within 14 days) by enteral administration.

10. Pharmaceutical composition according to the use of claim 9 for use in the treatment referred to in claim 9 **characterized in that** eradication of H. pylori is attained without administration of antibiotics during the course of therapy.

## Patentansprüche

1. Pharmazeutische Zusammensetzung zur Behandlung von Magen- und Zwölffingerdarmgeschwüren, welche mindestens einen PPI und mindestens ein Präbiotikum umfasst, wobei die Komponenten der Zusammensetzung in den folgenden Gewichtsprozenten enthalten sind:
- Protonenpumpeninhibitor 0,05-25%;
- Präbiotikum 40-95%;
- Exzipienten ad 100%,
wobei das Präbiotikum ausgewählt ist aus der Gruppe bestehend aus einem Di- oder Trisaccharid ausgewählt aus der Gruppe bestehend aus Lactulose, Lactosaccharose, Melibiose, Xylobiose, Stachyose und Raffinose; und/oder
einem Oligosaccharid ausgewählt aus der Gruppe bestehend aus F.ructooligosaccharid, Galactooligosaccharid, Maltooligosaccharid, Xylooligosaccharid, Isomaltooligosaccharid und Gentioligosaccharid; und/oder
einem Polysaccharid ausgewählt aus der Gruppe bestehend aus Arabinogalaktan, Pektin, Pullulan, Inulin und Lignin.

2. Pharmazeutische Zusammensetzung nach Anspruch 1, **dadurch gekennzeichnet, dass** sie einen Protonenpumpeninhibitor aus der folgenden Gruppe enthält: Omeprazol, Pantoprazol, Lansoprazol, Rabeprazol und Esomeprazol.

3. Pharmazeutische Zusammensetzung nach Anspruch 2, **dadurch gekennzeichnet, dass** sie den PPI Omeprazol in einer Menge von 10-120 mg enthält.

4. Pharmazeutische Zusammensetzung nach Anspruch 2, **dadurch gekennzeichnet, dass** sie den PPI Pantoprazol in einer Menge von 20-800 mg enthält.

5. Pharmazeutische Zusammensetzung nach Anspruch 2, **dadurch gekennzeichnet, dass** sie den PPI Lansoprazol in einer Menge von 10-600 mg enthält.

6. Pharmazeutische Zusammensetzung nach Anspruch 2, **dadurch gekennzeichnet, dass** sie den PPI Rabeprazol in einer Menge von 10-200 mg enthält.

7. Pharmazeutische Zusammensetzung nach Anspruch 2, **dadurch gekennzeichnet, dass** sie den PPI Esomeprazol in einer Menge von 20-240 mg enthält.

8. Pharmazeutische Zusammensetzung nach einem der Ansprüche 1 bis 7, **dadurch gekennzeichnet, dass** es sich bei der Dosierungsform der Zusammensetzung um eine Suspension zur oralen Verabreichung, eine Lösung zur oralen Verabreichung oder Kapseln, Tabletten, Pulver, Beutelchen, Pellets, Granulate für die orale Verabreichung handelt.

9. Pharmazeutische Zusammensetzung nach einem der Ansprüche 1 bis 8 zur Verwendung bei der Behandlung von Magen- und Zwölffingerdarmgeschwüren, wenn die Ausmerzung von H. pylori im Verlauf der Therapie in Patienten (mindestens einmal täglich, vor dem Essen, mindestens innerhalb von 14 Tagen) durch enterale Verabreichung erzielt wird.

10. Pharmazeutische Zusammensetzung gemäß der Verwendung nach Anspruch 9 zur Verwendung bei der Behandlung, auf die in Anspruch 9 verwiesen wurde, **dadurch gekennzeichnet, dass** die Ausmerzung von H. pylori ohne die Verabreichung von Antibiotika im Verlauf der Therapie erzielt wird.

## Revendications

1. Composition pharmaceutique pour le traitement de l'ulcère gastrique et duodénal incluant au moins un IPP et au moins un prébiotique avec les teneurs suivantes pour les composants de la composition en % massiques :
- inhibiteur de la pompe à protons entre 0,05 et 25 % ;
- prébiotique entre 40 et 95 % ;
- excipients jusqu'à 100 %,
où le prébiotique est choisi dans le groupe constitué par les suivants :
un di- ou trisaccharide choisi dans le groupe constitué par le lactulose, le lactosucrose, le mélibiose, le xylobiose, le stachyose et le raffinose ; et/ou un oligosaccharide choisi dans le groupe constitué par le fructo-oligosaccharide, le galacto-oligosaccharide, le malto-oligosaccharide, le xylo-oligosaccharide, l'isomalto-oligosaccharide et le gentioligosaccharide ; et/ou un polysaccharide choisi dans le groupe constitué par l'arabinogalactane, la pectine, le pullulane, l'inuline et la lignine.

2. Composition pharmaceutique selon la revendication 1, **caractérisée en ce qu'**elle contient un inhibiteur de la pompe à protons choisi dans le groupe constitué par les suivants : oméprazole, pantoprazole, lansoprazole, rabéprazole et ésoméprazole.

3. Composition pharmaceutique selon la revendication 2, **caractérisée en ce qu'**elle contient l'IPP oméprazole dans une quantité comprise entre 10 et 120 mg.

4. Composition pharmaceutique selon la revendication 2, **caractérisée en ce qu'**elle contient l'IPP pantoprazole dans une quantité comprise entre 20 et 800 mg.

5. Composition pharmaceutique selon la revendication 2, **caractérisée en ce qu'**elle contient l'IPP lansoprazole dans une quantité comprise entre 10 et 600 mg.

6. Composition pharmaceutique selon la revendication 2, **caractérisée en ce qu'**elle contient l'IPP rabéprazole dans une quantité comprise entre 10 et 200 mg.

7. Composition pharmaceutique selon la revendication 2, **caractérisée en ce qu'**elle contient l'IPP ésoméprazole dans une quantité comprise entre 20 et 240 mg.

8. Composition pharmaceutique selon l'une quelconque des revendications 1 à 7, **caractérisée en ce que** la forme galénique de la composition est une suspension pour administration orale, une solution pour administration orale, ou des capsules, comprimés, poudres, sachets, billes, granules pour administration orale.

9. Composition pharmaceutique selon l'une quelconque des revendications 1 à 8 pour utilisation dans le traitement de l'ulcère gastrique et duodénal, où l'éradication de *H. pylori* est obtenue en cours de traitement chez les patients (au moins une fois par jour, avant les repas, au moins, dans un délai de 14 jours) par administration entérale.

10. Composition pharmaceutique selon l'utilisation de la revendication 9 pour utilisation dans le traitement désigné dans la revendication 9, **caractérisée en ce que** l'éradication de *H. pylori* est obtenue sans administration d'antibiotiques en cours de traitement.
